(19) Europäisches Patentamt
European Patent Office
Office européen des brevets

(11) Numéro de publication : **0 482 993 A1**

(12) # DEMANDE DE BREVET EUROPEEN

(21) Numéro de dépôt : **91402810.5**

(51) Int. Cl.$^5$ : **C07F 9/659,** A61K 31/675

(22) Date de dépôt : **22.10.91**

(30) Priorité : **23.10.90 FR 9013103**

(43) Date de publication de la demande :
**29.04.92 Bulletin 92/18**

(84) Etats contractants désignés :
**AT BE CH DE DK ES FR GB GR IT LI LU NL SE**

(71) Demandeur : **PIERRE FABRE MEDICAMENT**
**45, Place Abel Gance**
**F-92100 Boulogne (FR)**

(72) Inventeur : **Duflos, Alain**
**Chemin du Landou**
**F-81100 Castres (FR)**
Inventeur : **Patoiseau, Jean-François**
**7, rue Jules Ferry**
**F-81100 Castres (FR)**
Inventeur : **Bigg, Dennis**
**122 avenue de Lavaur**
**F-81100 Castres (FR)**
Inventeur : **Kiss, Robert**
**114 avenue Emilie de Villeneuve**
**F-81100 Castres (FR)**

(74) Mandataire : **Ahner, Francis et al**
**CABINET REGIMBEAU, 26, avenue Kléber**
**F-75116 Paris (FR)**

(54) **Dérivés N,O spirocycliques de cyclotriphosphazènes, leur préparation et leur application en thérapeutique.**

(57) La présente invention se rapporte à des dérivés de formule générale I :

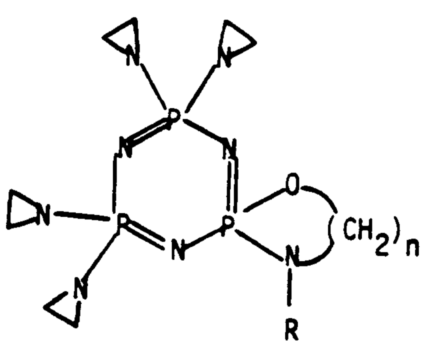

I

Elle concerne également les compositions pharmaceutiques comprenant à titre de principe actif au moins un de ces composés de formule générale I.

EP 0 482 993 A1

La présente invention, réalisée au Centre de Recherche PIERRE FABRE MEDICAMENT, a pour objet de nouveaux composés chimiques, leur procédé de préparation et leur application en tant que médicaments.

Les nouveaux composés chimiques, revendiqués par la demanderesse répondent à la formule générale I :

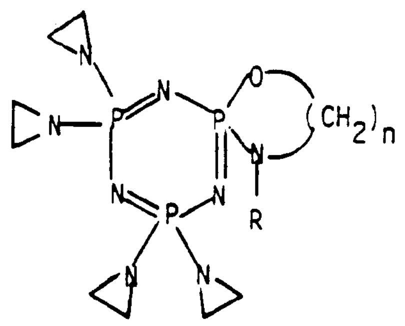

I

dans laquelle :

– R représente l'hydrogène, un radical alcoyle linéaire. ramifié ou cyclique en $C_{1-6}$, un radical phényle ou un radical phénvl alcoyle $\Phi(CH_2)_m$-, m étant compris entre 1 et 4 et, à titre d'exemple non limitatif H, Me, cyclohexyle, phényle, benzyle, phénéthyle.

– n = 2, 3 ou 4.

La présente invention concerne également l'utilisation des composés de formule générale I à titre de médicament et les compositions pharmaceutiques renfermant ce médicament. Les compositions pharmaceutiques selon la présente invention peuvent utiliser un composé de formule générale I éventuellement en association avec un ou plusieurs principes actifs.

Les procédés de synthèse des composés de formule générale I font aussi partie de la présente invention.

Synthèse des composés de formule générale I.

Les composés de la présente invention sont obtenus à partir de l'hexachlorocyclotriphosphazène II via les intermédiaires tétrachlorés III.

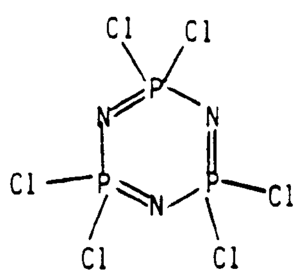

II

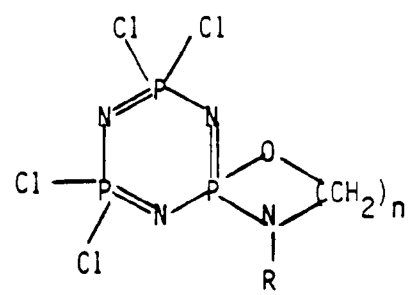

III

Les composés III peuvent être obtenus selon les méthodes décrites dans la littérature, notamment par :

– S.S. KRISHNAMURTHY et Coll., J.C.S. Dalton 1980, p. 840-844

– V. CHANDRASEKHAR et Coll., Inorg. Nucl. Chem. Letters, 1981, vol. 17, n°5-6, p. 181-185.

Ces méthodes consistent à traiter l'hexachlorocyclotriphosphazène ($N_3P_3Cl_6$) par l'hydroxy amine IV en présence d'un capteur d'acide chlorhydrique tel que la triéthylamine dans un solvant tel que le tétrahydrofurane.

$R-NH-(CH_2)_n-OH$ IV

Il est également possible d'accéder aux intermédiares III en conduisant la réaction en milieu biphasique tel que THF/soude.

L'aziridination des intermédiaires III pour obtenir les composés de la présente invention est effectuée par l'aziridine dans un solvant tel que le THF en présence d'un capteur d'acide chlorhydrique tel que la triéthylamine, à titre d'exemple.

L'exemple suivant illustre l'invention sans en limiter la portée.

4-méthyl-7,7,9,9-tétra-1-aziridinyl-7,7,9,9-tétrahydro-1-oxa-4,6,8,10-tétraaza-55-7,9-triphosphaspiro-[4, 5]-déca-5,7,9-triène

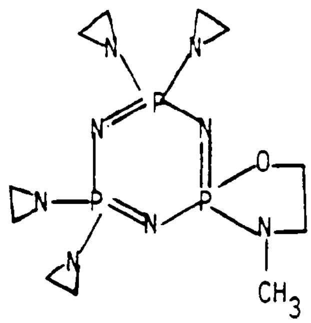

a) Dans un réacteur d'un litre, on introduit 8 g (106 mmoles) de N méthyl amino-2 éthanol, 300 ml de THF et 266 ml de soude aqueuse N.

Sous agitation violente, à 25°C, on introduit en une fois le $N_3P_3Cl_6$ (la température est maintenue en dessous de 35°C). Après 1 heure à 30°C, la phase aqueuse est saturée par du chlorure de sodium, puis après décantation, extraite à l'éther éthylique.

Les 2 phases organiques, groupées, sont évaporées à sec. Le résidu, repris par un minimum de chlorure de méthylène, est percolé à travers une colonne de silice (20 g), élué par un mélange chlorure de méthylène/acétate d'éthyle 1/1.

Après évaporation, le résidu est agité dans l'hexane pendant 30 mn.

On obtient, après filtration et séchage à 40°C sous vide, 26 g d'intermédiaire tétrachloré.

Rendement 70 % - F = 137°C.

b) Le composé obtenu ci-dessus (17,5 g - 50 mmoles) est mis en solution dans 250 ml de THF contenant de la triéthylamine. (30 g - 300 mmoles). On ajoute, à -10°C en 15 mn, l'aziridine (12,9 g - 300 mmoles) diluée dans le THF (50 ml).

Après agitation pendant 1 heure à O°C et 48 heures à 25°C, on filtre les cristaux de chlorhydrate et évapore la moitié du THF. La solution obtenue est percolée sur une colonne de silice (50 g) et éluée au THF (150 ml).

Après évaporation à sec et recristallisation du résidu du THF, on obtient le composé 1 (10,5 g).

Rendement 56 % - F = 170°C.

A titre d'exemple, afin d'illustrer l'invention sans en limiter la portée, le tableau ci-après résume les caractéristiques de produits synthétisés de manière analogue à l'exemple ci-dessus.

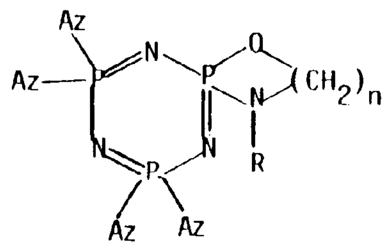

| N° | R | A | Solv. Recrist. | Rdt | Formule brute | P.M. | F°C | RMN $^{31}P$ PAz$_2$ | RMN $^{31}P$ P Spiro | J |
|---|---|---|---|---|---|---|---|---|---|---|
| 1 | $CH_3$ | $(CH_2)_2$ | THF | 56 % | $C_{11}H_{23}N_8OP_3$ | 376,28 | 170 | 39.55 | 32.37 | 39.04<br>41.5 |
| 2 | H | $(CH_2)_2$ | ACOEt-EI*1-1 | 59 % | $C_{10}H_{21}N_8OP_3$ | 362,25 | 112 | 39,68<br>39,78 | 36.33 | 43,3<br>37,3 |
| 3 | H | $(CH_2)_3$ | THF | 67 % | $C_{11}H_{23}N_8OP_3$ | 376,28 | 145 | 38,45 | 19.04 | 38,24 |
| 4 | cyclohexyl | $(CH_2)_3$ | EI | 47 % | $C_{17}H_{33}N_8OP_3$ | 458.43 | 101 | 38,34 | 19.48 | 39,95 |
| 5 | benzyl | $(CH_2)_2$ | ACOEt-EI 1-1 | 41 % | $C_{17}H_{27}N_8OP_3$ | 452,38 | 144 | 39,7 | 31.67 | 40,2 |
| 6 | benzyl | $(CH_2)_3$ | ACOEt-EI 1-1 | 50 % | $C_{18}H_{29}N_8OP_3$ | 466.41 | 133 | 38,56 | 20.89 | 39,35 |
| 7 | phénéthyl | $(CH_2)_2$ | ACOEt-EI 1-1 | 60 % | $C_{18}H_{29}N_8OP_3$ | 466.41 | 124 | 39,5 | 31.48 | 40,35 |
| 8 | phénéthyl | $(CH_2)_3$ | ACOEt-EI 1-1 | 35 % | $C_{19}H_{31}N_8OP_3$ | 480.43 | 116 | 38,4 | 20.42 | 38,68 |
| 9 | H | $(CH_2)_4$ | ACOEt | 40 % | $C_{12}H_{25}N_8OP_3$ | 390,31 | 129 | | | |

* EI = éther isopropylique

## EXPERIMENTATIONS

Divers essais pharmacologiques et toxicologiques ont été effectués sur les composés objets de la présente invention.

### 1- Activité pharmacologique

a) Cytotoxicité in vitro

L'activité cytotoxique in vitro a été évaluée par la détermination de l'$IC_{50}$ (concentration pour laquelle on obtient 50 % d'inhibition de croissance cellulaire) sur les cellules leucémiques L 1210 de la souris.
L'$IC_{50}$ est évaluée à l'aide d'un compteur cellulaire.

| Composé | $IC_{50}$ (µM) |
|---|---|
| 1 | 9 |
| 2 | 8,7 |
| 3 | 2,3 |
| 4 | 9,9 |
| 5 | 5,8 |
| 6 | 5,8 |
| 7 | 7,8 |
| 8 | 6,5 |

b) In vivo

L'activité antitumorale in vivo a été évaluée sur le modèle P 388 de la souris. Ce modèle correspond à des cellules leucémiques greffées i.p. au jour J.
La drogue est administrée en doses répétées à J1, J3, J5.

Nous représentons l'indice "T/C" correspondant à la survie (%) des animaux traités par rapport aux animaux contrôles.
Les "T/C" des produits vont de 130 à > 300.

### 2 - Toxicité

Les composés testés présentent une toxicité modérée, aux doses testées, au niveau des paramètres hématologiques et de l'évolution pondérale.

### 3 - Applications thérapeutiques

Compte tenu de leurs propriétés pharmacologiques, les composés de la présente invention peuvent être utilisés en thérapeutique humaine dans le traitement de la pathologie cancéreuse.

Les préparations pharmaceutiques contenant ces principes actifs peuvent être mises en forme pour l'administration par voie orale, intra-veineuse ou intra-péritonéale.

Il est également possible d'y associer d'autres principes actifs pharmaceutiquement et thérapeutiquement acceptables.

## Revendications

**1 -** Composés nouveaux correspondant à la formule générale I

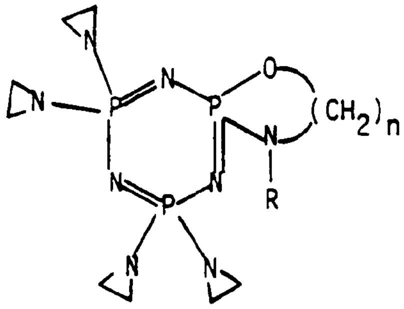

I

dane laquelle :

– R représente l'hydrogène, un radical alcoyle linéaire, ramifié ou cyclique en $C_{1-6}$, un radical phényle ou un radical phényl alcoyle $\Phi(CH_2)_m$-, m étant compris entre 1 et 4 et, à titre d'exemple non limitatif H, Me, cyclohexyle, phényle, benzyle, phénéthyle.

– n = 2, 3 ou 4.

**2 -** Composés de formule générale I selon la revendication 1 caractérisés en ce que R est choisi parmi hydrogène, méthyle, cyclohexyle, benzyle, phénéthyle.

**3 -** Procédé de préparation des composés selon les revendications 1, 2, 3 caractérisé en ce que l'on traite les composés intermédiares III par l'aziridine en présence d'un capteur d'acide dans un solvant organique tel que le THF.

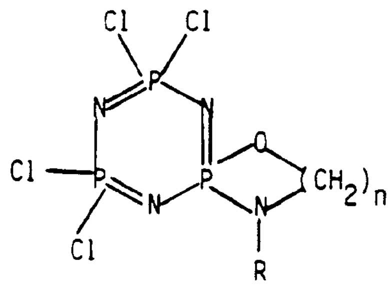

III

**4 -** A titre de médicaments nouveaux, utiles par exemple dans le traitement des maladies cancéreuses, les composés définis selon l'une des revendications 1 à 3.

**5 -** Compositions pharmaceutiques caractérisées en ce qu'elles contiennent comme principe actif au moins un composé défini selon l'une des revendications 1 à 3.

**6 -** Compositions pharmaceutiques selon la revendication 6, caractérisées en ce qu'elles renferment en outre un autre principe actif.

Office européen des brevets

# RAPPORT DE RECHERCHE EUROPEENNE

Numero de la demande

EP 91 40 2810

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| Y | EP-A-0 112 743 (CENTRE NATIONAL DE LA RECHERCHE SCIENTIFIQUE)<br>* le document en entier *<br>--- | 1-6 | C07F9/659<br>A61K31/675 |
| Y | INORGANICA CHIMICA ACTA, vol. 67, 1982, pages L5 -L7, Elsevier Sequoia, CH; G. GUERCH et al.: " A through route for the synthesis of pure 2,2,4,4-tetrakis(aziridinyl)-6,amino-6,methoxycyclophos-phaza-1,3,5-triene gem-N3P3AZ4(NH2)(OCH3)" *en entier*<br>----- | 1-6 | |
| | | | DOMAINES TECHNIQUES RECHERCHES (Int. Cl.5 ) |
| | | | C07F |

Le présent rapport a été établi pour toutes les revendications

| Lieu de la recherche | Date d'achèvement de la recherche | Examinateur |
|---|---|---|
| LA HAYE | 22 JANVIER 1992 | BESLIER L.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)